# EUROPÄISCHE PATENTSCHRIFT

(11) **EP 0 816 343 B1**
(45) Veröffentlichungstag und Bekanntmachung des Hinweises auf die Patenterteilung: **14.12.2005**
(21) Anmeldenummer: 97109739.9
(22) Anmeldetag: 16.06.1997
(51) Int. Cl.: C07D 233/76, C07D 233/78, C07B 57/00

(54) **Verfahren zur Herstellung chiraler, nicht racemischer (4-Aryl-2,5-dioxoimidazolidin-1-yl)-essigsäuren**
Process for the preparation of chiral, non-racemic (4-aryl-2,5-dioxoimidazolidin-1-yl)-acetic acids
Procédé pour la préparation d'acides (4-aryl-2,5-dioxoimidazolidin-1-yl)-acétiques chiraux, non racémiques

(30) Priorität: 20.06.1996 DE 19624604
(43) Veröffentlichungstag der Anmeldung: 07.01.1998
(73) Patentinhaber: Sanofi-Aventis Deutschland GmbH, 65929 Frankfurt am Main (DE)
(72) Erfinder: Holla, Wolfgang, Dr., 65779 Kelkheim (DE); Beck, Gerhard, Dr., 60320 Frankfurt (DE); Kammermeier, Bernhard, Dr., 80687 München (DE); Kulitzscher, Berndt, Dl., 97839 Steinmark (DE); Michalowsky, Jürgen, 65779 Kelkheim (DE)

(56) Entgegenhaltungen:
- EP-A- 0 132 854
- EP-A- 0 182 279
- EP-A- 0 468 592
- EP-A- 0 529 835
- WO-A-95/14008
- WO-A-96/33976
- CHEMICAL ABSTRACTS, vol. 62, no. 5, 1.März 1965 Columbus, Ohio, US; abstract no. 5268bcd, Spalte 5268; XP002042157 & J. Med. Chem., 1965, 8 (1), 117-120 und 120-122
- H. U. STILZ ET AL.: "From peptides to heterocyclic peptide mimetics - design and synthesis of an orally active fibrinogen receptor antagonist for the prevention of thrombosis" BULLETIN DES SOCIETES CHIMIQUES BELGES, Bd. 105, Nr. 10-11, Oktober 1996, Seiten 711-9, XP002042155
- N. BUCKLEY ET AL.: "A reinvestigation of the alkylation of 5-(p-hydroxyphenyl)-5-phenylhydantoin" CLINICA CHIMICA ACTA, Bd. 62, 1975, Seiten 73-8, XP002042308
- J. FALBE ET AL. (HRSG.): "Römpp Chemie Lexikon, 9. Auflage, Bd. 5, Pl-S" 1992 , G. THIEME VERLAG , STUTTGART, DE XP002042156 * Seiten 3753-3754, Stichwort "Racemattrennung", besonders Seite 3753, rechte Spalte, Abbildung *

## Beschreibung

Die vorliegende Erfindung betrifft ein Verfahren zur Herstellung chiraler, nicht racemischer Verbindungen der allgemeinen Formel I, 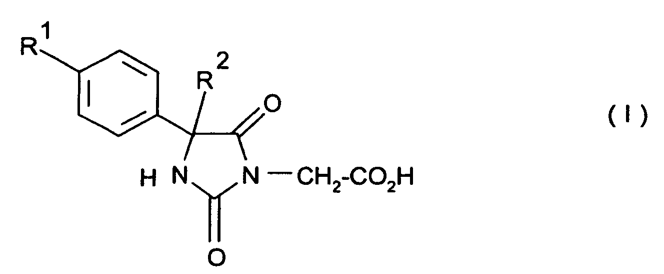 die wertvolle Zwischenprodukte für die Herstellung von pharmazeutischen Wirkstoffen darstellen, bei dem zur Racematspaltung ein Salz aus der racemischen Verbindung der allgemeinen Formel I und einem chiralen, nicht racemischen phenylsubstituierten Aminoalkohol gebildet wird. Sie betrifft weiterhin Verbindungen der allgemeinen Formel I und Ester davon.

In der PCT-Anmeldung PCT/EP 94/03491 sind Hydantoinderivate beschrieben, die die Zell-Zell-Adhäsion, insbesondere z. B. die Thrombocytenaggregation, hemmen. Unter anderem werden darin Verbindungen offenbart, die eine (4-Aryl-2,5-dioxoimidazolidin-1-yl)acetyl-Einheit enthalten. Die pharmakologische Wirksamkeit dieser Substanzen hängt unter anderem von der Konfiguration an C-4 des 2,5-Dioxoimidazolidin- oder Hydantoinringes ab. Die Herstellung von Wirkstoffen mit einheitlicher Konfiguration an C-4 des Hydantoinringes erfolgt nach der PCT-Anmeldung PCT/EP94/03491 in aufwendiger Weise, indem auf der Stufe des Wirkstoffes eine chromatographische Auftrennung eines Stereoisomerengemisches durchgeführt wird, das hinsichtlich C-4 des Hydantoinringes ein Gemisch der R-Form und der S-Form ist.

In der deutschen Patentanmeldung 195 15 177 sowie der PCT-Anmeldung PCT/EP96/01572 werden Hydantoinderivate, darunter auch Hydantoinessigsäuren der allgemeinen Formel I, beschrieben die als Zwischenprodukte bei der Synthese der in der PCT-Anmeldung PCT/EP94/03491 beschriebenen Wirkstoffe eingesetzt werden können. Enantiomerenreine Hydantoinessigsäuren, die sich für die Herstellung von Wirkstoffen mit einheitlicher Konfiguration an C-4 des Hydantoinringes eignen, sind gemäß der PCT-Anmeldung PCT/EP96/01572 aber nur nach aufwendigen Verfahren in vielstufigen Synthesen erhältlich, beispielsweise indem eine Carbonylverbindung in einer Bucherer-Reaktion zu einem Hydantoin umgesetzt wird, dieses zur Aminosäure hydrolysiert wird, die Aminosäure verestert wird, mit dem Aminosäureester eine Racematabspaltung durchgeführt wird, die enantiomerenreine Verbindung mit einem Isocyanatoessigsäureester umgesetzt wird und das erhaltene Produkt abschließend unter sauren Bedingungen zur Hydantoinessigsäure cyclisiert wird.

Diese bekannten Methoden für die Herstellung der in der PCT-Anmeldung PCT/EP94/03491 beschriebenen Wirkstoffe mit einheitlicher Konfiguration an C-4 des Hydantoinringes sind wegen ihrer geringen Gesamtausbeute und der aufwendigen Verfahren für eine Wirkstoffproduktion im technischen Maßstab nicht akzeptabel. Es besteht daher Bedarf an einer einfachen synthetischen Methode für die Herstellung der gewünschten Wirkstoffe.

Einige racemische Hydantoinessigsäuren der allgemeinen Formel I und deren Ethylester, unter anderem die Verbindungen, in denen R¹ in der allgemeinen Formel I für Chlor und R² für Methyl steht, und ihre pharmakologische Aktivität sind bereits von M. B. Winstead und C. R. Hamel, J. Med. Chem. 8 (1965), 120-122, beschrieben worden.

Überraschenderweise wurde gefunden, daß sich nicht racemische Hydantoinessigsäuren der allgemeinen Formel I in einfacher Weise und in hohen optischen und chemischen Ausbeuten durch eine Racematspaltung auf der Stufe der bequem zugänglichen racemischen Verbindungen der allgemeinen Formel I herstellen lassen, bei der aus der racemischen Verbindung der allgemeinen Formel I und einem chiralen, nicht racemischen phenylsubstituierten Aminoalkohol ein Salz gebildet wird (Grundsätzliches zur Racematspaltung wird zum Beispiel im Römpp Chemie Lexikon (J. Falbe und M. Regitz (Herausgeber), 9. Auflage, Band 5, 3753-3754, Georg Thieme Verlag Stuttgart und New York, 1992) diskutiert). Mit den dadurch erhältlichen nicht racemischen Hydantoinessigsäuren der allgemeinen Formel I, in denen an C-4 also einheitlich oder überwiegend die R-Konfiguration oder die S-Konfiguration vorliegt, lassen sich dann gemäß den Angaben in der PCT-Anmeldung PCT/EP96/01572 die gewünschten Wirkstoffen herstellen. Die in die Racematspaltung eingesetzten racemischen Verbindungen der allgemeinen Formel I, also die Enantiomerengemische, lassen sich nach oder analog zu bekannten Methoden, so z.B. auch gemäß den Angaben in der PCT-Anmeldung PCT/EP94/03491, herstellen.

Gegenstand der vorliegenden Erfindung ist somit ein Verfahren zur Herstellung chiraler, nicht racemischer Verbindungen der allgemeinen Formel I, 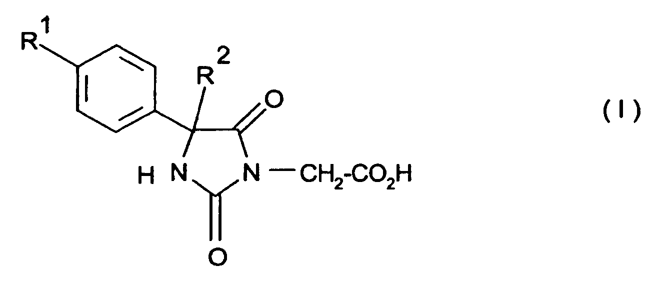 in der
R¹ für Wasserstoff, Fluor, Chlor, Brom, Iod, Cyan, Nitro oder Hydroxy steht und
R² für Wasserstoff, Fluor, (C₁-C₇)-Alkyl, Phenyl-(C₁-C₇)-alkyl oder (C₃-C₈)-Cycloalkyl steht,
dadurch gekennzeichnet, daß eine Racematspaltung mit der racemischen Verbindung der allgemeinen Formel I durchgeführt wird, bei der aus der racemischen Verbindung der allgemeinen Formel I und einem chiralen, nicht racemischen phenylsubstituierten Aminoalkohol ein Salz gebildet wird.

Alkylreste können geradkettig oder verzweigt sein. Dies gilt auch, wenn sie substituiert sind. Beispiele für Alkylreste sind Methyl, Ethyl, n-Propyl, Isopropyl, n-Butyl, Isobutyl, sec-Butyl, tert.-Butyl, n-Pentyl, Isopentyl, Neopentyl, n-Hexyl, Isohexyl, n-Heptyl. Bevorzugte Alkylreste sind Methyl, Ethyl, n-Propyl, Isopropyl und tert.-Butyl, besonders bevorzugt sind Methyl und Ethyl.

In Phenyl-(C₁-C₇)-alkyl-Resten kann sich die Phenylgruppe in beliebiger Position in der Alkyl-Gruppe befinden. Beispiele für Phenyl-(C₁-C₇)-alkyl-Reste sind Benzyl, 1-Phenylethyl, 2-Phenylethyl, 3-Phenylpropyl, 4-Phenylbutyl und 6-Phenylhexyl. Bevorzugte Reste sind Phenyl-(C₁-C₄)-alkyl-Reste, ein besonders bevorzugter Rest ist Benzyl.

Beispiele für Cycloalkylreste sind Cyclopropyl, Cyclobutyl, Cyclopentyl, Cyclohexyl, Cycloheptyl und Cyclooctyl. Cycloalkylreste können auch durch einen oder mehrere Alkylreste, insbesondere Methylreste, substituiert sein. Ein bevorzugter Cycloalkylrest ist der Cyclopropylrest.

R¹ steht bevorzugt für Chlor, Brom, Iod, Cyan oder Hydroxy, besonders bevorzugt für Chlor, Brom oder Cyan.

R² steht bevorzugt für Wasserstoff, Fluor, (C₁-C₄)-Alkyl, insbesondere Methyl oder Ethyl, Phenyl-(C₁-C₄)-alkyl, insbesondere Benzyl, oder (C₃-C₇)-Cycloalkyl, insbesondere Cyclopropyl, besonders bevorzugt für Wasserstoff, Methyl oder Ethyl, ganz besonders bevorzugt für Methyl oder Ethyl, darüber hinaus bevorzugt für Methyl.

Bevorzugt ist es weiterhin, wenn gleichzeitig R¹ für Chlor, Brom oder Cyan steht und R² für Methyl oder Ethyl steht.

Die Herstellung der in die Racematspaltung eingesetzten racemischen Verbindungen der allgemeinen Formel I kann nach dem Fachmann geläufigen Methoden erfolgen. Beispielsweise kann von einer Carbonylverbindung der allgemeinen Formel II 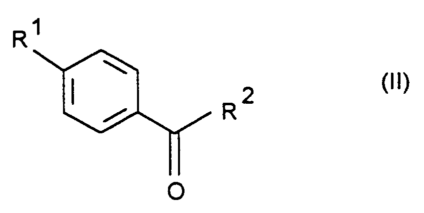 ausgegangen werden, in der
R¹ für Wasserstoff, Fluor, Chlor, Brom, Iod, Cyan, Nitro oder Hydroxy steht und
R² für Wasserstoff, (C₁-C₇)-Alkyl, Phenyl-(C₁-C₇)-alkyl oder (C₃-C₈)-Cycloalkyl steht.

Bevorzugt wird von Carbonylverbindungen der allgemeinen Formel II ausgegangen, in der R¹ für Wasserstoff, Cyan, Brom, Chlor, lod oder Hydroxy, besonders bevorzugt für Brom oder Hydroxy, steht. Bevorzugte Bedeutungen von R² in der allgemeinen Formel II sind, Wasserstoff, (C₁-C₄)-Alkyl, insbesondere Methyl und Ethyl, Phenyl-(C₁-C₄)-alkyl, insbesondere Benzyl, und (C₃-C₇)-Cycloalkyl, insbesondere Cyclopropyl, besonders bevorzugte Bedeutungen von R² in der allgemeinen Formel II sind Methyl und Ethyl.

Die Verbindungen der allgemeinen Formel II können unter den bekannten Bedingungen der Bucherer-Reaktion (H.T. Bucherer, V.A. Lieb, J. Prakt. Chem. 141 (1934), 5-43) durch Umsetzung mit Kaliumcyanid und Ammoniumcarbonat in die racemischen Hydantoine der allgemeinen Formel III, 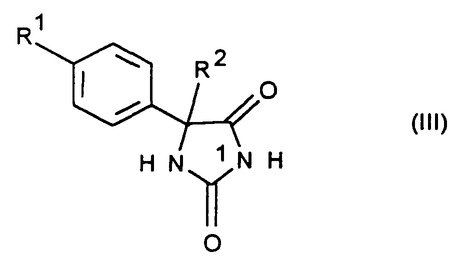 in der R¹ und R² wie für die allgemeine Formel II angegeben definiert sind, überführt werden. Die Verbindungen der allgemeinen Formel III können analog literaturbekannten Methoden (siehe z.B. Houben-Weyl, Methoden der Organischen Chemie, Band XI/1, S. 81ff, oder M. Orena et al., J. Org. Chem. 57 (1992), 6532) in Gegenwart von Basen, beispielsweise Alkoholaten oder Alkalimetallhydroxiden oder -carbonaten wie Kaliumhydroxid oder Kaliumcarbonat, mit Halogenessigsäureestern der allgemeinen Formel IV, 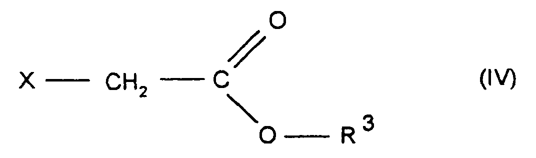 in der X für Chlor, Brom oder Iod, insbesondere Chlor oder Brom, steht und R³ beispielsweise für (C₁-C₆)-Alkyl, insbesondere Methyl, Ethyl oder tert.-Butyl, oder Benzyl steht, an N-1 zu den Estern der allgemeinen Formel V 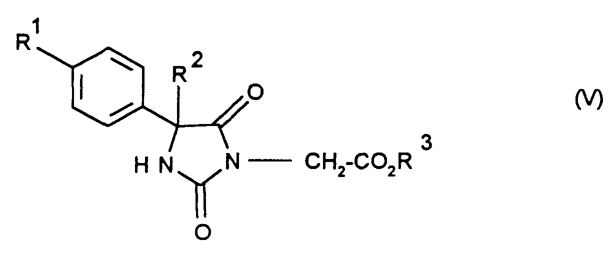 alkyliert werden, in der R¹ und R² wie für die allgemeine Formel III angegeben definiert sind und R³ wie für die allgemeine Formel IV angegeben definiert ist. Eine besonders bevorzugte Verbindung der allgemeinen Formel IV ist der Chloressigsäuremethylester. Bei der Alkylierung können auch katalytisch wirkende Substanzen, z.B. Iodide wie Kaliumiodid oder Natriumiodid bei der Verwendung von Chlor- oder Bromessigsäureestern, zugesetzt werden. Die Ester der allgemeinen Formel V können dann nach dem Fachmann geläufigen Methoden, z.B. mit wäßrigen Mineralsäuren wie Salzsäure oder wäßrigen Alkalihydroxidlösungen wie Natronlauge, in die racemischen Hydantoinessigsäuren der allgemeinen Formel I überführt werden. Die Überführung der Ester der allgemeinen Formel V in die Säuren kann direkt im Anschluß an die Alkylierung ohne Isolierung des Esters vorgenommen werden, die Ester können aber auch zwischendurch isoliert werden.

Verbindungen der allgemeinen Formel I, in der R² für Fluor steht, können aus Verbindungen, in denen R² für Wasserstoff steht, nach üblichen, dem Fachmann bekannten Fluorierungsmethoden hergestellt werden (siehe z.B. Nachr. Chem. Tech. Lab. 38 (1990), 40).

In den racemischen Verbindungen der allgemeinen Formel I kann gewünschtenfalls auch noch eine Umwandlung des Substituenten R¹ durchgeführt werden. So können beispielsweise Verbindungen der allgemeinen Formel I, in der R¹ für Halogen steht, durch einen analog bekannter Verfahren durchgeführten Halogen-Cyan-Austausch, z.B. einen Brom-Cyan-Austausch, in Verbindungen der allgemeinen Formel I überführt werden, in der R¹ für Cyan steht (N. Chatani und T. Hanafusa, J. Org. Chem. 51 (1986), 4714; J.R. Dalton und S.L. Regen, J. Org. Chem. 44 (1979), 4443), und Verbindungen, in denen R¹ für Hydroxy steht, können direkt oder nach Umwandlung der Hydroxygruppe z.B. in die Methylsulfonyloxygruppe oder die Trifluormethylsulfonyloxygruppe durch einen Cyanaustausch in Verbindungen überführt werden, in denen R¹ für Cyan steht (M.R.I. Chambers und D.A. Widdowson, J. Chem. Soc. Perkin Trans. I (1989), 1365; V. Percec et al., J. Org. Chem 60 (1995), 6895; und darin zitierte Literatur), und Verbindungen, in denen R¹ für Wasserstoff steht, können direkt in Verbindungen überführt werden, in denen R¹ für Cyan steht (G. Lohaus, Chem. Ber. 100 (1967), 2719). Solche Umwandlungen können auch in den nicht racemischen Verbindungen der allgemeinen Formel I durchgeführt werden.

Die für die Racematspaltung durchzuführende Salzbildung aus der racemischen Verbindung der allgemeinen Formel I und dem chiralen, nicht racemischen phenylsubstituierten Aminoalkohol erfolgt gemäß der üblichen Vorgehensweise durch Vereinigung der beiden Komponenten in einem Lösungs-, Verdünnungs- oder Dispergiermittel. Die anschließende eigentliche Racematspaltung, also die Abtrennung oder Anreicherung oder Abreicherung einer der beiden enantiomeren Formen der Verbindung der allgemeinen Formel I, erfolgt dann unter Ausnutzung der unterschiedlichen Eigenschaften, die das Salz aus der R-Form der Verbindung der allgemeinen Formel I und dem chiralen, nicht racemischen phenylsubstituierten Aminoalkohol einerseits und das Salz aus der S-Form der Verbindung der allgemeinen Formel I und dem chiralen, nicht racemischen phenylsubstituierten Aminoalkohol andererseits haben. Bei der Vereinigung der beiden Komponenten kann die racemische Verbindung der allgemeinen Formel I vorgelegt werden und der phenylsubstituierte Aminoalkohol zudosiert werden oder umgekehrt, und es können auch beide Komponenten gleichzeitig in das Reaktionsgefäß dosiert werden.

Die bei der Racematspaltung eingesetzten chiralen, nicht racemischen phenylsubstituierten Aminoalkohole können gesättigt oder ungesättigt sein und unsubstituierte oder substituierte Phenylreste, enthalten. Sie können primäre, sekundäre und tertiäre Aminogruppen enthalten, und Aminogruppen können auch Teil eines Ringsystems sein. Beispiele für geeignete chirale phenylsubstituierte Aminoalkohole sind (+)-Ephedrin, (-)-Ephedrin, (+)-Norephedrin, (-)-Norephedrin, (+)-Phenylalaninol oder (-)-Phenylalaninol.

Die Wahl des Lösungs-, Verdünnungs- oder Dispergiermittels, im folgenden zusammenfassend als Lösungsmittel bezeichnet, in dem die racemische Verbindung der allgemeinen Formel I und der chirale, nicht racemische phenylsubstituierte Aminoalkohol zur Salzbildung vereinigt werden, hängt vom Einzelfall ab, also von der jeweiligen Kombination aus phenylsubstituiertem Aminoalkohol und Verbindung der allgemeinen Formel I und den Eigenschaften ihrer Salze sowie von der für die Racematspaltung vorgesehenen Vorgehensweise. Geeignete Lösungsmittel sind beispielsweise Wasser und organische Lösungsmittel wie Alkohole, z.B. Methanol, Ethanol, Propanol und Isopropanol, Ether, z.B. tert.-Butylmethylether, Dioxan, Tetrahydrofuran und Mono- und Dimethylether des Ethylenglykols und des Diethylenglykols, Ketone, z.B. Aceton und Butanon, Ester, z.B. Ethylacetat und tert.-Butylacetat, und Kohlenwasserstoffe und halogenierte Kohlenwasserstoffe, z.B. Toluol und Methylenchlorid. Es können auch Gemische aus zwei oder mehr Lösungsmittel eingesetzt werden, beispielsweise Gemische aus Wasser und Ethanol, aus Wasser und Methanol, aus Isopropanol und tert.-Butylmethylether oder aus Wasser und Ethylacetat, z.B. in Form von mit Wasser gesättigten Ethylacetat. Vielfach ist es zweckmäßig in einem wäßrig-organischen Lösungsmittel zu arbeiten, also in einem Gemisch aus Wasser und einem oder mehreren organischen Lösungsmitteln, oder in Wasser.

Die racemische Verbindung der allgemeinen Formel I und der chirale, nicht racemische phenylsubstituierte Aminoalkohol können zur Salzbildung im Molverhältnis 1:1 vereinigt werden, es kann aber auch eine der beiden Komponenten im Unterschuß oder im Überschuß eingesetzt werden. Bevorzugt werden 0.5 bis 1 Mol phenylsubstituierter Aminoalkohol auf 1 Mol racemische Verbindung der allgemeinen Formel I eingesetzt. Wird der phenylsubstituierte Aminoalkohol im Unterschuß eingesetzt, so kann es von Vorteil sein, eine nicht chirale Hilfsbase zuzusetzen, die den nicht durch den phenylsubstituierten Aminoalkohol in das Salz überführten Teil der Verbindung der allgemeinen Formel I ganz oder teilweise neutralisiert. Bevorzugt werden in diesem Fall bis zu 0.5 Mol Hilfsbase auf 1 Mol racemische Verbindung der allgemeinen Formel I eingesetzt. Als Hilfsbasen eignen sich z.B. Alkalihydroxide wie Lithium-, Natrium- oder Kaliumhydroxid.

Die Salzbildung wird im allgemeinen bei Temperaturen von -15°C bis 100°C, bevorzugt 0°C bis 80°C, durchgeführt.

Bei der nach der Salzbildung zur Spaltung des Racemats durchzuführenden Abtrennung oder Anreicherung bzw. Abreicherung einer der beiden enantiomeren Formen der Verbindung der allgemeinen Formel I aus dem ursprünglichen racemischen Gemisch kann ausgenutzt werden, daß die Bildung eines der beiden diastereomeren Salze, also des Salzes aus der R-Form der Verbindung der allgemeinen Formel I und dem chiralen, nicht racemischen phenylsubstituierten Aminoalkohol einerseits und des Salzes aus der S-Form der Verbindung der allgemeinen Formel I und dem chiralen, nicht racemischen phenylsubstituierten Aminoalkohol andererseits, bevorzugt erfolgt und/oder daß Löslichkeitsunterschiede zwischen diesen beiden Salzen bestehen. Es kann beispielsweise aus dem eingesetzten Lösungsmittel nur eines der beiden Salze ausfallen oder eines der beiden Salze in stärkeren Maße ausfallen als das andere. Das ausgefallene Salz kann dann z.B. durch Filtrieren oder Zentrifugieren isoliert werden und dieses Salz und/oder die Mutterlauge weiterverarbeitet werden. Zur Abtrennung oder Anreicherung bzw. Abreicherung einer Form kann aber beispielsweise auch eine Verteilung zwischen zwei nicht mischbaren flüssigen Phasen bzw. eine Extraktion erfolgen. Vorzugsweise geht man so vor, daß bei und/oder nach der Salzbildung eine Kristallisation eines der beiden diastereomeren Salze oder eines Salzgemisches, in dem eines der beiden Salze überwiegt, stattfindet, also eine fraktionierende Kristallisation. Für die Herstellung eines bestimmten Enantiomeren der allgemeinen Formel I kann es dabei von Vorteil sein, die Bedingungen so zu wählen, daß nicht das gewünschte Enantiomere als Salz mit dem chiralen phenylsubstituierten Aminoalkohol ausfällt, sondern das unerwünschte, und das erwünschte zunächst in der Mutterlauge verbleibt.

Bei der bevorzugten Vorgehensweise mit fraktionierender Kristallisation enthält das Reaktionsgemisch der Salzbildung vorzugsweise 2.5 bis 40 Gewichtsprozent, besonders bevorzugt 10 bis 30 Gewichtsprozent, an racemischer Verbindung der allgemeinen Formel I, bezogen auf das Gesamtgewicht des Reaktionsgemisches. Die Temperatur während des Kristallisationsvorgangs beträgt im allgemeinen -15°C bis 100°C, bevorzugt 0°C bis 80°C. Sie kann während der Kristallisation verändert werden, z.B. kann zunächst bei einer höheren Temperatur die Kristallisation eingeleitet werden und dann die Temperatur gesenkt werden. Vielfach ist es günstig, zum Schluß eine Temperatur von -5°C bis 30°C einzustellen.

Das bei der fraktionierenden Kristallisation ausfallende Salz bzw. die Mutterlauge enthält die eine bzw. die andere enantiomere Form der Verbindung der allgemeinen Formel I oft bereits in einer hohen optischen Reinheit, die für die Wirkstoffsynthese ausreicht. Wird das im ausfallenden Salz enthaltene Enantiomer in einer höheren Reinheit gewünscht, so kann nach der Isolierung dieses Salzes z.B. durch Filtrieren oder Zentrifugieren eine weitere Anreicherung z.B. durch Umkristallisation angeschlossen werden. Diese Umkristallisation, die eine weitere fraktionierende Kristallisation darstellt, kann einmal oder mehrmals durchgeführt werden, bis die im Einzelfall gewünschte optische Reinheit erreicht ist. Für die Lösungsmittel, die bei der Umkristallisation der Salze aus den Verbindungen der allgemeinen Formel I und den chiralen, nicht racemischen phenylsubstituierten Aminoalkoholen eingesetzt werden können, gelten die obigen Ausführungen zu den Lösungsmitteln bei der Salzbildung entsprechend. Vorzugsweise werden bei der Umkristallisation Wasser, Alkohole wie Methanol, Ethanol oder Isopropanol, oder Gemische aus diesen, z.B. Gemische aus Wasser und Ethanol, eingesetzt.

Beim Umkristallisieren liegt die Konzentration des eingesetzten Salzes vorzugsweise bei 2.5 bis 40 Gewichtsprozent, besonders bevorzugt bei 10 bis 30 Gewichtsprozent, bezogen auf das Gesamtgewicht des Umkristallisationsansatzes. Die Temperatur beim Umkristallisieren liegt im allgemeinen bei -15°C bis 100°C, bevorzugt bei 0°C bis 80°C. Vielfach ist es günstig, zum Schluß vor der Isolierung der ausgefallenen Kristalle z.B. durch Filtrieren oder Zentrifugieren eine Temperatur von -5°C bis 30°C einzustellen.

Wird das Enantiomere, das in Form seines Salzes mit dem chiralen phenylsubstituierten Aminoalkohol in der Mutterlauge der nach der Salzbildung erfolgten Kristallisation enthalten ist, in einer höheren optischen Reinheit als der zunächst erreichten gewünscht, so kann z.B. die Mutterlauge teilweise oder vollständig eingeengt werden und das erhaltene Salz gemäß den vorstehenden Ausführungen umkristallisiert werden.

Zur Freisetzung der enantiomerenreinen Verbindungen der allgemeinen Formel I mit der gewünschten optischen Reinheit aus ihren Salzen mit den chiralen phenylsubstituierten Aminoalkoholen können die direkt nach der Salzbildung oder nach Umkristallisation isolierten Salze nach der üblichen Vorgehensweise z.B. in Wasser oder in einem organischen Lösungsmittel oder in einem Gemisch aus Wasser und einem organischen Lösungsmittel gelöst oder suspendiert werden und mit einer starken Säure versetzt werden, z.B. mit einer Mineralsäure wie Salzsäure, Salpetersäure, Phosphorsäure oder Schwefelsäure, insbesondere mit einer wäßrigen Mineralsäure. Die resultierenden freien Carbonsäuren der allgemeinen Formel I können dann je nach den Gegebenheiten des Einzelfalls z.B. durch Filtrieren, Zentrifugieren, Phasentrennung oder Extrahieren isoliert werden. Bevorzugt wird die Freisetzung der Carbonsäuren der allgemeinen Formel I in Wasser oder einem Gemisch aus Wasser und einem organischen Lösungsmittel, z.B. in einem Wasser-Ethylacetat-Gemisch, durchgeführt, mit wäßriger Mineralsäure ein pH von ca. 0 bis 2 eingestellt und die Carbonsäure durch Phasentrennung und/oder Extraktion, z.B. mit Ethylacetat, und anschließendes Einengen der organischen Phasen und Trocknen des Rückstandes isoliert. Analog, also z.B. durch Ansäuern und Extraktion, zur beschriebenen Freisetzung aus den isolierten Salzen können auch die Carbonsäuren der allgemeinen Formel I gewonnen werden, die bei der Salzbildung in der Mutterlauge eines auskristallisierten Salzes verblieben sind. Dies gilt sowohl für Carbonsäuren, die in Form ihrer Salze mit den chiralen phenylsubstituierten Aminoalkoholen in der Mutterlauge verblieben sind, als auch für Salze mit gegebenenfalls zugesetzten Hilfsbasen.

Aus der bei der Freisetzung der Verbindungen der allgemeinen Formel I nach deren Isolierung verbleibenden sauren Lösung kann der für die Racematspaltung verwendete chirale, nicht racemische phenylsubstituierte Aminoalkohol wiedergewonnen werden. Dazu kann so vorgegangen werden, daß die saure Lösung mit einer starken Base, z.B. einem Alkalihydroxid wie Natronlauge oder Kalilauge, auf einen pH-Wert von 11 oder größer eingestellt wird und der dann in Form der freien Base vorliegende phenylsubstituierte Aminoalkohol isoliert wird, z.B. durch Extrahieren aus einer wäßrigen Lösung oder Suspension mit einem organischen Lösungsmittel wie Ethylacetat, Trocknen und Einengen der Extrakte. Der wiedergewonnene phenylsubstituierte Aminoalkohol kann vor der erneuten Verwendung in einer Racematspaltung noch z.B. durch Digerieren mit einem Lösungsmittel oder durch Umkristallisieren gereinigt werden.

Nach dem erfindungsgemäßen Verfahren werden auf einfache, im technischen Maß stab leicht durchführbare Weise in hohen chemischen Ausbeuten chirale, nicht racemische Verbindungen der allgemeinen Formel I erhalten, die sehr gute optische Reinheiten aufweisen und sich daher hervorragend für die Herstellung der gewünschten pharmazeutischen Wirkstoffe mit einheitlicher Konfiguration an C-4 des Hydantoinringes eignen. Die Weiterverarbeitung zu pharmazeutischen Wirkstoffen kann z.B. nach den in den PCT-Anmeldungen PCT/EP94/03491 und PCT/EP96/01572 für die racemischen und die nicht racemischen Verbindungen der allgemeinen Formel I beschriebenen Verfahren erfolgen, auf die hier Bezug genommen wird.

Gegenstand der vorliegenden Erfindung sind weiterhin Verbindungen der allgemeinen Formel I als solche, die in racemischer Form die Ausgangssubstanzen für das oben beschriebene erfindungsgemäße Verfahren sind bzw. die in nicht racemischer Form bei der Durchführung dieses Verfahrens erhalten werden, und die wertvolle Zwischenprodukte für die Herstellung von pharmazeutischen Wirkstoffen sind. Die nicht racemischen Verbindungen der allgemeinen Formel I können dabei in Form reiner Enantiomerer oder in Form von Gemischen der Enantiomeren in beliebigen Verhältnissen vorliegen (wobei bei einem Verhältnis von 1 : 1 die racemische Verbindung vorliegt). Insbesondere sind Gegenstand der vorliegenden Erfindung Verbindungen der allgemeinen Formel Ia, 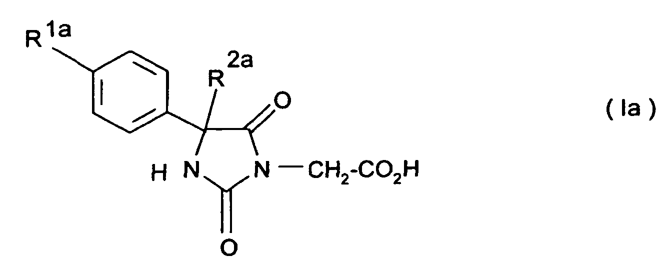 in der
R^{1a} für Fluor, Chlor, Brom oder Hydroxy steht und
R^{2a} für (C₁-C₄)-Alkyl oder Benzyl steht,
in racemischer oder nicht racemischer Form, wobei die in Form der reinen Enantiomeren vorliegende Verbindung der allgemeinen Formel la ausgeschlossen ist, in der R^{1a} für Brom und R^{2a} für Methyl steht, und die in racemischer Form vorliegende Verbindung der allgemeinen Formel la ausgeschlossen ist, in der R^{1a} für Chlor und R^{2a} für Methyl steht.

Die obigen Erläuterungen zum erfindungsgemäßen Verfahren und zu den Verbindungen der allgemeinen Formel I gelten für die Verbindungen der allgemeinen Formel la entsprechend. Beispiele für die für R^{2a} stehende (C₁-C₄)-Alkylgruppe in der allgemeinen Formel Ia sind Methyl, Ethyl, n-Propyl, Isopropyl, n-Butyl, Isobutyl, sec-Butyl und tert.-Butyl. Bevorzugt steht R^{2a} für Methyl oder Ethyl, besonders bevorzugt für Methyl. Verbindungen der allgemeinen Formel la, in der R^{1a} für Brom steht, sind speziell in racemischer Form Gegenstand der vorliegenden Erfindung. Eine bevorzugte Verbindung, die als solche Gegenstand der vorliegenden Erfindung ist, ist die Verbindung der allgemeinen Formel Ia, in der R^{1a} für Brom und R^{2a} für Methyl steht, in racemischer Form, d. h. also die Verbindung der Formel Ib 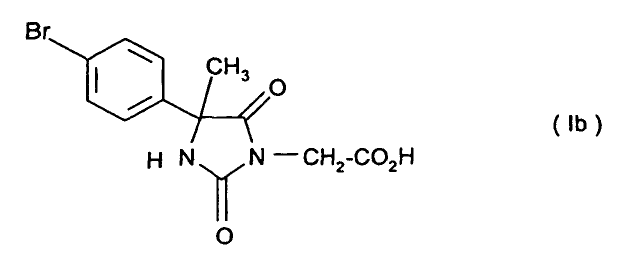 in racemischer Form, die als Ausgangsmaterial in das oben beschriebene erfindungsgemäße Verfahren eingesetzt werden kann.

Die Herstellung der Verbindungen der allgemeinen Formel la und der Verbindung der Formel Ib kann z. B. erfolgen, indem man wie oben erläutert die entsprechenden racemischen Hydantoine der allgemeinen Formel III mit Halogenessigsäureestern der allgemeinen Formel IV zu den racemischen Verbindungen der allgemeinen Formel Va 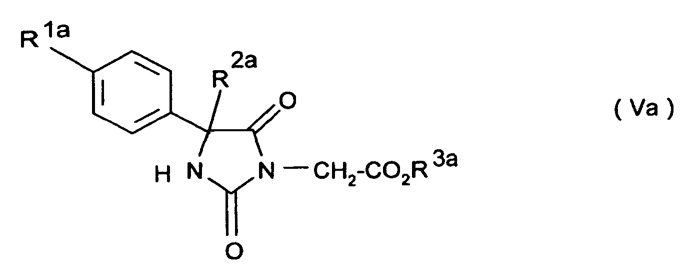 umsetzt, in der
R^{1a} für Fluor, Chlor, Brom oder Hydroxy steht,
R^{2a} für (C₁-C₄)-Alkyl oder Benzyl steht und
R^{3a} beispielsweise für (C₁-C₆)-Alkyl oder Benzyl steht,
und diese racemischen Ester wie oben beschrieben beispielsweise mit wäßrigen Mineralsäuren oder Alkalihydroxidlösungen zu den Säuren der allgemeinen Formel la bzw. zur Säure der Formel Ib in der racemischen Form verseift, aus denen dann gemäß dem erfindungsgemäßen Verfahren die Säuren der allgemeinen Formel la bzw. die Säure der Formel Ib in der nicht racemischen Form erhältlich sind. Auch die Verbindungen der allgemeinen Formel Va in racemischer Form sind Gegenstand der vorliegenden Erfindung, mit Ausnahme der Verbindung, in der R^{1a} für Chlor, R^{2a} für Methyl und R^{3a} für Ethyl steht. Auch für diese gelten die obigen Erläuterungen entsprechend. Bevorzugt haben R^{1a} und R^{2a} in der allgemeinen Formel Va die für die allgemeine Formel la angegebenen bevorzugten Bedeutungen. R^{3a} in der allgemeinen Formel Va steht bevorzugt für (C₁-C₄)-Alkyl, insbesondere Methyl, Ethyl oder tert.-Butyl, oder Benzyl. Bevorzugte Verbindungen sind diejenigen Verbinungen der allgemeinen Formel Va in racemischer Form, in der R^{1a} für Brom, R^{2a} für Methyl und R^{3a} für (C₁-C₄)-Alkyl, insbesondere Methyl oder Ethyl, steht, d. h. also die racemischen Verbindungen der allgemeinen Formel Vb, 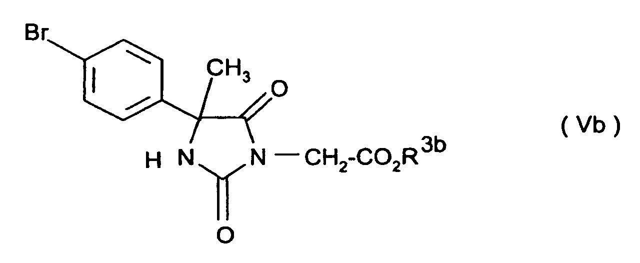 in der R^{3b} für (C₁-C₄)-Alkyl, insbesondere Methyl oder Ethyl, steht. Die Verbindungen der allgemeinen Formeln Va und Vb sind ebenfalls wertvolle Zwischenprodukte für pharmazeutische Wirkstoffe. Die Verbindungen der allgemeinen Formeln Va und Vb können wie oben erläutert nach ihrer Herstellung aus den Verbindungen der allgmeinen Formeln III und IV isoliert werden, sie können aber ebenso auch ohne Isolierung direkt zu den Säuren der allgemeinen Formel la bzw. zur Säure der Formel Ib verseift werden. Erfolgt die Verseifung unter alkalischen Bedingungen, so entstehen zunächst Salze dieser Säuren, die natürlich ebenfalls von der vorliegenden Erfindung umfaßt werden.

### Beispiele

Die Produkte wurden durch ihre ¹H-NMR-Spektren und Massenspektren identifiziert. Der Enantiomerenüberschuß (ee) einer Säure der allgemeinen Formel I an der (R)-Form bzw. der (S)-Form in den erhaltenen Produkten wurde durch Hochdruckflüssigkeitschromatographie (HPLC) bestimmt (Säule: S,S-Whelk-01 (250 mm x 4 mm) der Fa. E. Merck, Darmstadt; Detektor: UV 240/254 mm; Elutionsmittel: n-Hexan + Ethanol + Eisessig (90+10+ 1 Volumenteile); Fluß: 1 ml/min; Temperatur: 40°C). Die in den Beispielen erhaltenen Salze wurden direkt durch HPLC untersucht. Die Bestimmung der absoluten Konfiguration der Verbindungen der allgemeinen Formel I erfolgte über die unabhängige Herstellung der nicht racemischen Verbindungen aus den entsprechenden literaturbekannten optisch reinen Aminosäuren (vgl. PCT-Anmeldung PCT/EP96/01572).

### Beispiel 1

### (R,S)-4-Phenyl-4-methyl-2,5-dioxoimidazolidin

21.0 g Acetophenon, 15.8 g Kaliumcyanid und 54.0 g Ammoniumcarbonat werden in 340 ml Wasser-Ethanol (1:1) im Autoklaven 8 h bei 10 bar auf 110°C erhitzt. Anschließend verdünnt man mit 500 ml Wasser und gibt unter Eiskühlung vorsichtig (starkes Schäumen, Bildung von Cyanwasserstoff) 90 ml konz. Salzsäure hierzu. Das ausgefallene Produkt wird abfiltriert, mit Wasser gewaschen und getrocknet. Ausbeute: 29.1 g (87.5 %) der racemischen Titelverbindung. Schmp. 194 bis 195°C.

### Beispiel 2

### (R,S)-4-(4-Bromphenyl)-4-methyl-2,5-dioxoimidazolidin

Im Autoklaven werden 29.4 ml Ethanol und 29.4 ml Wasser vorgelegt und nacheinander mit 5.8 g 4-Bromacetophenon, 2.48 g Kaliumcyanid und 8.4 g Ammoniumcarbonat versetzt. Das Reaktionsgemisch wird 8 h bei 8 bis 9 bar auf 110°C erhitzt. Nach Abkühlen auf Raumtemperatur verdünnt man mit 30 ml Wasser und stellt durch vorsichtige Zugabe von ca. 27 ml halbkonz. Salzsäure einen pH-Wert von 3 bis 4 ein. Der entstehende weiße Niederschlag wird abfiltriert, mit wenig Wasser gewaschen und im Trockenschrank bei 50°C getrocknet. Ausbeute: 7.7 g (98 %) der racemischen Titelverbindung. Schmp. 275°C.

### Beispiel 3

### (R,S)-4-(4-Cyanphenyl)-4-methyl-2,5-dioxoimidazolidin

65.0 g 4-Cyanacetophenon, 43.2 g Kaliumcyanid und 132.0 g Ammoniumcarbonat werden in 700 ml Wasser-Ethanol (1:1) im Autoklaven 8 h bei 10 bar (Stickstoff) auf 110°C erhitzt. Anschließend verdünnt man mit 350 ml Wasser und säuert vorsichtig mit ca. 460 ml halbkonz. Salzsäure bis zu einem pH-Wert von 3.5 an. Das ausgefallene Produkt wird abfiltriert, mit Wasser gewaschen und getrocknet. Ausbeute: 58 g (61 %) der racemischen Titelverbindung. Schmp. 206°C.

### Beispiel 4

### (R,S)-(4-(4-Bromphenyl)-4-methyl-2,5-dioxoimidazolidin-1-yl)essigsäure

10.58 g (R,S)-4-(4-Bromphenyl)-4-methyl-2,5-dioxoimidazolidin werden bei Raumtemperatur in 25 ml N-Methylpyrrolidon vorgelegt und mit 5.87 g gemahlenem Kaliumcarbonat und 5.45 g Chloressigsäuremethylester versetzt. Das Reaktionsgemisch wird 2 h auf 50°C erwärmt, dann auf 25°C abgekühlt und mit 250 ml Wasser verdünnt. Durch Zugabe von 8 bis 9 ml konz. Natronlauge wird ein pH-Wert von 11 eingestellt, dann wird 30 min bei Raumtemperatur und weitere 30 min bei 40 bis 50°C gerührt. Die erhaltene klare farblose Lösung wird auf 25°C abgekühlt, mit 13 bis 15 ml konz. Salzsäure auf einen pH-Wert von 1 bis 1.5 angesäuert und weitere 60 min bei 15 bis 20°C nachgerührt. Das ausgefallene kristalline Produkt wird abfiltriert, mit wenig Wasser neutral gewaschen und bei 60°C im Vakuum getrocknet. Ausbeute: 10.5 g (83 %) der racemischen Titelverbindung. Schmp. 240 bis 241 °C.

### Beispiel 5

### (R,S)-(4-(4-Bromphenyl)-4-methyl-2,5-dioxoimidazolidin-1-yl)essigsäure

10.0 g (R,S)-4-(4-Bromphenyl)-4-methyl-2,5-dioxoimidazolidin werden bei Raumtemperatur in 50 ml N-Methylpyrrolidon vorgelegt und mit 4,74 g Kalium-tert.-butylat und 4.7 ml Bromessigsäureethylester versetzt. Das Reaktionsgemisch wird 2.5 h auf 120°C erwärmt, dann auf 25°C abgekühlt und auf 200 ml Wasser gegeben. Nach Sättigen mit Natriumchlorid wird mit insgesamt 100 ml Ethylacetat extrahiert. Die vereinigten organischen Phasen werden eingeengt und der Rückstand wird mit 50 ml konz. Salzsäure 1 h auf 90 bis 100°C erhitzt. Das auskristallisierte Produkt wird abfiltriert, mit Wasser gewaschen und im Vakuum getrocknet. Ausbeute: 9.5 g (79 %) der racemischen Titelverbindung. Schmp. 239 bis 241 °C.

### Beispiel 6

### (R,S)-(4-(4-Cyanphenyl)-4-methyl-2,5-dioxoimidazolidin-1-yl)essigsäure

34.4 g (R,S)-4-(4-Cyanphenyl)-4-methyl-2,5-dioxoimidazolidin werden bei Raumtemperatur in 80 ml N-Methylpyrrolidon vorgelegt und mit 24.32 g gemahlenem Kaliumcarbonat und 16.32 ml Chloressigsäuremethylester versetzt. Das Reaktionsgemisch wird 2 h bei 50 bis 60°C gerührt, dann auf 25°C abgekühlt und mit 800 ml Wasser verdünnt. Mit konz. Natronlauge wird ein pH-Wert von 11 eingestellt, dann wird 30 min bei Raumtemperatur und weitere 30 min bei 40 bis 50°C gerührt. Nach Abkühlen auf 25°C wird mit 344 ml 2 N Salzsäure auf einen pH-Wert von 1 angesäuert und dreimal mit ca. 250 ml Ethylacetat extrahiert. Die vereinigten organischen Phasen werden mit gesättigter Natriumchloridlösung gewaschen, mit Natriumsulfat getrocknet und im Vakuum eingeengt. Man erhält 51.2 g eines Öls, das aus Diisopropylether kristallisiert wird. Ausbeute: 36.3 g (83 %) der racemischen Titelverbindung. Schmp. 227 bis 229°C.

### Beispiel 7

### (R, S)-(4-(4-Cyanphenyl)-4-methyl-2,5-dioxoimidazolidin-1-yl)essigsäure

12.5 g (R,S)-(4-(4-Bromphenyl)-4-methyl-2,5-dioxoimidazolidin-1-yl)essigsäure und 15.4 g Kupfer(I)cyanid werden in 65 ml Dimethylformamid 8 h auf 155°C erwärmt, dann läßt man den Ansatz unter Rühren über Nacht auf ca. 25°C abkühlen. Die aus der anfänglich weißen Suspension erhaltene grünliche Lösung wird mit 195 ml Wasser versetzt, und der pH-Wert wird mit konz. Salzsäure von 3 auf pH = 1 bis 1.5 eingestellt. Man rührt 30 min, gibt 8 g Celite®-Filterhilfsmittel und 50 g Natriumchlorid zu, rührt weitere 15 min und filtriert. Die wäßrige Phase wird dreimal mit je 40 ml Ethylacetat extrahiert. Die vereinigten organischen Phasen werden dreimal mit je 25 ml gesättigter Natriumchloridlösung gewaschen. Die Natriumchloridlösungen werden verworfen. Die organische Phase wird mit 100 ml verdünnter Natronlauge versetzt und das Gemisch über eine Seitz-Filterschicht filtriert. Die organische Phase wird abgetrennt und verworfen, die alkalische wäßrige Phase wird mit konzentrierter Salzsäure auf einen pH-Wert von 1 bis 1.5 angesäuert. Das zunächst ölig ausfallende Produkt kristallisiert nach kurzer Zeit. Man rührt 1 h bei 10°C nach, filtriert das Produkt ab, wäscht mit Wasser neutral und trocknet. Ausbeute: 7.5 g (72 %) der racemischen Titelverbindung. Schmp. 224 bis 226°C.

### Beispiel 8

### (S)-(4-(4-Bromphenyl)-4-methyl-2,5-dioxoimidazolidin-1-yl)essigsäure

10.8 g (R,S)-(4-(4-Bromphenyl)-4-methyl-2,5-dioxoimidazolidin-1-yl)essigsäure und 5.0 g (R)-Phenylalaninol werden in 90 ml Isopropanol 15 min bei 80°C gerührt. Man kühlt auf 20 bis 25°C ab, filtriert das auskristallisierte Produkt ab und erhält 10.3 g des (R)-Phenylalaninol-Salzes, das aus 110 ml Ethanol umkristallisiert wird. Nach zweistündigen Rühren bei 5°C wird das auskristallisierte Produkt abfiltriert und getrocknet.
Ausbeute: 4.4 g des (R)-Phenylalaninol-Salzes der Titelsäure.

Zur Freisetzung der freien Säure suspendiert man das Salz in 44 ml Wasser, säuert mit verdünnter Salzsäure auf einen pH-Wert von 1 an, filtriert den resultierenden Niederschlag ab, digeriert ihn mit Wasser und trocknet. Ausbeute: 2.85 g der Titelsäure mit 66 % ee der (S)-Form (HPLC).
[α]²⁰_{D} = +18° (c = 1; 2.15 N ethanolische Chlorwasserstofflösung).

### Beispiel 9

### (R)-Phenylalaninol-Salz der (S)-(4-(4-Cyanphenyl)-4-methyl-2,5-dioxoimidazolidin-1-yl)essigsäure

30.0 g (R,S)-(4-(4-Cyanphenyl)-4-methyl-2,5-dioxoimidazolidin-1-yl)essigsäure werden bei 40°C in 600 ml Ethanol gelöst und mit 9.96 g (R)-Phenylalaninol versetzt. Man läßt den Ansatz 16 h bei 0 bis 5°C rühren und filtriert das ausgefallene Produkt ab. Ausbeute: 14.8 g des Titelsalzes mit 82 % ee der (S)-Säure (HPLC). Schmp. 185-187°C.

### Beispiel 10

### (R)-Phenylalaninol-Salz der (S)-(4-(4-Cyanphenyl)-4-methyl-2,5-dioxo-imidazolidin-1-yl)essigsäure

3.8 g des nach Beispiel 9 erhaltenen Salzes werden in 105 ml Ethanol in der Siedehitze gelöst. Man läßt abkühlen, rührt 1.5 h bei 0 bis 5°C, filtriert das ausgefallene Produkt ab und trocknet es. Ausbeute: 3.0 g des Titelsalzes mit 97.5 % ee der (S)-Säure (HPLC). Schmp. 193 bis 195°C.

### Beispiel 11

### (S)-Phenylalaninol-Salz der (R)-(4-(4-Cyanphenyl)-4-methyl-2,5-dioxoimidazolidin-1-yl)essigsäure

25 g (R,S)-(4-(4-Cyanphenyl)-4-methyl-2,5-dioxoimidazolidin-1-yl)essigsäure werden bei 20 bis 25°C in einer Mischung aus 150 ml Isopropanol und 150 ml tert.-Butylmethylether mit 13.8 g (S)-Phenylalaninol versetzt. Nach Rühren über Nacht wird das ausgefallene Produkt abfiltriert und getrocknet. Ausbeute:
9.3 g des Titelsalzes mit 71.3 % ee der (R)-Säure (HPLC). Durch zweimaliges Umkristallisieren aus Isopropanol/Aceton wird in einer Ausbeute von 3 g das Titelsalz mit 99.4 % ee der (R)-Säure erhalten.

### Beispiel 12

### (R)-Phenylalaninol-Salz der (S)-(4-(4-Cyanphenyl)-4-methyl-2,5-dioxoimidazolidin-1-yl)essigsäure

Zu 15.1 g (R)-Phenylalaninol in 150 ml Wasser werden bei 20°C 27.3 g (R,S)-(4-(4-Cyanphenyl)-4-methyl-2,5-dioxoimidazolidin-1-yl)essigsäure unter Rühren zugegeben. Das Gemisch wird 10 min bei 20°C, dann 1 h bei 60°C gerührt, wobei eine klare Lösung entsteht. Dann wird innerhalb von 3 h auf 20°C abgekühlt und 15 h bei dieser Temperatur nachgerührt. Das ausgefallene Produkt wird abgesaugt, mit 20 ml Wasser und 40 ml Aceton gewaschen und getrocknet. Ausbeute: 18.9 g des Titelsalzes mit 89.8 % ee der (S)-Säure (HPLC). Schmp. 191 bis 194°C.

### Beispiel 13

### (R)-Phenylalaninol-Salz der (S)-4-(4-Cyanphenyl)-4-methyl-2,5-dioxoimidazolidin-1-yl)essigsäure

18.9 g des nach Beispiel 12 erhaltenen Salzes werden in 65 ml Wasser 1 h bei 60°C gerührt. Die Suspension wird in 3 h auf 20°C abgekühlt und 15 h bei dieser Temperatur nachgerührt. Das ausgefallene Produkt wird abgesaugt, mit 10 ml Wasser gewaschen und im Vakuum bei 40°C getrocknet. Ausbeute:
15.7 g des Titelsalzes mit 99.4 % ee der (S)-Säure (HPLC). Schmp. 199°C.
[α]²⁰_{D} = -18° (c=1; Methanol)

### Beispiel 14

### (S)-(4-(4-Cyanphenyl)-4-methyl-2,5-dioxoimidazolidin-1-yl)essigsäure

12.9 g des gemäß Beispiel 13 erhaltenen (R)-Phenylalaninol-Salzes der (S)-(4-(4-Cyanphenyl)-4-methyl-2,5-dioxoimidazolidin-1-yl)essigsäure werden in einer Mischung von 60 ml Wasser und 50 ml Ethylacetat vorgelegt. Durch Zugabe von halbkonz. Salzsäure wird ein pH-Wert von 1 eingestellt. Die wäßrige Phase wird abgetrennt und dient der Rückgewinnung von (R)-Phenylalaninol. Die organische Phase wird mit Wasser gewaschen und im Vakuum eingeengt. Der Rückstand wird mit 10 ml Toluol versetzt und zur Trockne eingedampft. Ausbeute: 8.2 g der Titelsäure mit 99.4 % ee der (S)-Form (HPLC).
[α]²⁰_{D} = +56° (c = 1; 29.2 %ige ethanolische Chlorwasserstofflösung).

### Beispiel 15

### Rückgewinnung von (R)-Phenylalaninol

Die nach Beispiel 14 erhaltene, das (R)-Phenylalaninol enthaltende wäßrige Phase wird mit konz. Natronlauge auf einen pH-Wert von 11 eingestellt und viermal mit je 10 bis 20 ml Ethylacetat extrahiert. Die vereinigten organischen Phasen werden mit Magnesiumsulfat getrocknet und im Vakuum eingedampft. Der Rückstand wird mit tert.-Butylmethylether digeriert und abgesaugt. Ausbeute: 3.8 g (R)-Phenylalaninol. Schmp. 92 bis 93°C.
[α]²⁰_{D} = +25.5° (c = 1.2; 1 N wäßrige Chlorwasserstofflösung)

### Beispiel 16

### (S)-Phenylalaninol-Salz der (R)-(4-(4-Cyanphenyl)-4-methyl-2,5-dioxoimidazolidin-1-yl)-essigsäure

19.6 g (R,S)-(4-(4-Cyanphenyl)-4-methyl-2,5-dioxoimidazolidin-1-yl)essigsäure werden in 90 ml Wasser auf 60°C erhitzt und nacheinander mit 5.44 g (S)-Phenylalaninol, 2 g Kaliumhydroxid und 20 ml Methanol versetzt. Man rührt 15 min bei 60°C, kühlt dann auf 0°C ab und rührt 15 min bei dieser Temperatur. Das ausgefallene Produkt wird abfiltriert und getrocknet. Ausbeute: 13.5 g des Titelsalzes mit 68.6 % ee der (R)-Säure (HPLC). Umkristallisation analog Beispiel 13 ergibt 12.2 g des Titelsalzes mit 100 % ee der (R)-Säure (HPLC). Schmp. 199 bis 200°C.

### Beispiel 17

### (S)-Phenylalaninol-Salz der (R)-(4-(4-Cyanphenyl)-4-methyl-2,5-dioxoimidazolidin-1-yl)essigsäure

19.6 g (R,S)-(4-(4-Cyanphenyl)-4-methyl-2,5-dioxoimidazolidin-1-yl)essigsäure werden in 90 ml Wasser auf 60°C erhitzt und nacheinander mit 5.44 g (S)-Phenylalaninol, 2 g Kaliumhydroxid und 10 ml Ethanol versetzt. Man rührt 15 min bei 60 bis 65°C, kühlt dann langsam auf 0°C ab und rührt 60 min bei dieser Temperatur. Das ausgefallene Produkt wird abfiltriert und getrocknet. Ausbeute: 13.4 g des Titelsalzes mit 72.2 % ee der (R)-Säure (HPLC). Umkristallisation analog Beispiel 13 ergibt 12.5 g des Titelsalzes mit 93.95 % ee der Säure (HPLC). Schmp. 199 bis 200°C.

### Beispiel 18

### (R)-Phenylalaninol-Salz der (S)-(4-(4-Cyanphenyl)-4-methyl-2,5-dioxoimidazolidin-1-yl)essigsäure

15.0 g (R,S)-(4-(4-Cyanphenyl)-4-methyl-2,5-dioxoimidazolidin-1-yl)essigsäure in 300 ml Ethylacetat und 11 ml Wasser werden bei 40°C mit 5.0 g (R)-Phenylalaninol versetzt. Man läßt langsam abkühlen und rührt über Nacht bei 0°C. Das ausgefallene Produkt wird abfiltriert und getrocknet. Ausbeute: 10.0 g des Titelsalzes mit 88.3 % ee der (S)-Säure (HPLC).

### Beispiel 19

### (R)-Phenylalaninol-Salz der (S)-(4-(4-Cyanphenyl)-4-methyl-2,5-dioxoimidazolidin-1-yl)essigsäure

Das nach Beispiel 18 erhaltene Produkt wird zweimal aus siedendem Isopropanol umkristallisiert, wobei man zunächst langsam unter Rühren auf 20 bis 25°C abkühlen läßt und dann über Nacht bei 5°C stehen läßt. Das ausgefallene Produkt wird abfiltriert und getrocknet. Ausbeute: 8.4 g des Titelsalzes mit 99.5 % ee der (S)-Säure (HPLC).

### Beispiel 20

### (S)-Phenylalaninol-Salz der (R)-(4-(4-Cyanphenyl)-4-methyl-2,5-dioxoimidazolidin-1-yl)essigsäure

19.6 g (R,S)-(4-(4-Cyanphenyl)-4-methyl-2,5-dioxoimidazolidin-1-yl)essigsäure werden in 70 ml Wasser auf 60°C erhitzt und mit 2 g Kaliumhydroxid, 5.44 g (S)-Phenylalaninol und 30 ml Ethanol versetzt. Man rührt 15 min bei 60°C, kühlt dann langsam auf 20 bis 25°C ab, rührt 16 h bei dieser Temperatur und kühlt dann auf 0°C. Das ausgefallene Produkt wird abfiltriert und im Vakuum bei 50°C getrocknet. Ausbeute: 12.4 g des Titelsalzes mit 86.7 % ee der (R)-Säure (HPLC). Schmp. 196 bis 197°C.

Durch 45 minütiges Rühren des so erhaltenen Salzes in 26 ml Wasser bei 60 bis 65°C, Abkühlen auf ca. 22°C, Rühren über Nacht, Abfiltrieren des ausgefallenen Produkts und Trocknen werden 11.4 g des Titelsalzes mit 98.5 % ee der (R)-Säure (HPLC) erhalten. Schmp. 199 bis 200°C.

### Beispiel 21

### (S)-Phenylalaninol-Salz der (R)-(4-(4-Cyanphenyl)-4-methyl-2,5-dioxoimidazolidin-1-yl)essigsäure

19.6 g (R,S)-(4-(4-Cyanphenyl)-4-methyl-2,5-dioxoimidazolidin-1-yl)essigsäure, 5.44 g (S)-Phenylalaninol und 2 g Kaliumhydroxid werden in 105 ml Wasser auf 60°C erhitzt, dann langsam auf 20 bis 25°C abgekühlt und 1 h bei dieser Temperatur gerührt. Das ausgefallene Produkt wird abfiltriert und getrocknet.
Ausbeute: 10.8 g des Titelsalzes mit 94.4 % ee der (R)-Säure (HPLC). Schmp. 199 bis 201 °C.

### Beispiel 22

### (+)-(1S,2R)-Ephedrin-Salz der (S)-(4-(4-Cyanphenyl)-4-methyl-2,5-dioxoimidazolidin-1-yl-essigsäure

7.1 g (R,S)-(4-(4-Cyanphenyl)-4-methyl-2,5-dioxoimidazolidin-1-yl)essigsäure werden in 40 ml Aceton auf ca. 45°C erhitzt und mit 2.13 g (+)-(1S,2R)-Ephedrin versetzt. Dann wird langsam auf 20 bis 25°C abgekühlt. Das ausgefallene Produkt wird abfiltriert und getrocknet. Ausbeute: 5.4 g des Titelsalzes mit 35 % ee der (S)-Säure (HPLC).

### Beispiel 23

### (+)-(1S,2R)-Ephedrin-Salz der (S)-(4-(4-Cyanphenyl)-4-methyl-2,5-dioxoimidazolidin-1-yl)essigsäure

Das nach Beispiel 22 erhaltene Produkt wird in 40 ml Ethanol unter Erhitzen gelöst und dann auf 20 bis 25°C abgekühlt. Das ausgefallene Produkt wird abfiltriert und getrocknet. Ausbeute: 2.7 g des Titelsalzes mit 98.1 % ee der (S)-Säure (HPLC).

### Beispiel 24

### (+)-(1S,2R)-Ephedrin-Salz der (R,S)-(4-(4-Cyanphenyl)-4-methyl-2,5-dioxoimidazolidin-1-yl)essigsäure

7.1 g (R,S)-(4-(4-Cyanphenyl)-4-methyl-2,5-dioxoimidazolidin-1-yl)essigsäure werden in 200 ml Methylenchlorid mit 2.13 g (+)-(1S,2R)-Ephedrin versetzt und 4 h bei 20 bis 25°C gerührt. Das ausgefallene Produkt wird abfiltriert und getrocknet.
Ausbeute: 8.8 g des Titelsalzes mit ≤ 1 % ee der Säure (HPLC).

### Beispiel 25

### (+)-(1S,2R)-Ephedrin-Salz der (S)-(4-(4-Cyanphenyl)-4-methyl-2,5-dioxoimidazolidin-1-yl)essigsäure

Das nach Beispiel 24 erhaltene Produkt wird in 40 ml Ethanol unter Erhitzen gelöst und dann auf 20 bis 25°C abgekühlt. Das ausgefallene Produkt wird abfiltriert und getrocknet. Ausbeute: 3.6 g des Titelsalzes mit 94.8 % ee der (S)-Säure (HPLC).

### Beispiel 26

### (S)-(4-(4-Cyanphenyl)-4-methyl-2,5-dioxoimidazolidin-1-yl)essigsäure

3 g des gemäß Beispiel 23 erhaltenen (+)-(1S,2R)-Ephedrin-Salzes der (S)-(4-(4-Cyanphenyl)-4-methyl-2,5-dioxoimidazolidin-1-yl)essigsäure werden in einer Mischung aus 15 ml Wasser und 15 ml Ethylacetat vorgelegt. Durch Zugabe von halbkonz. Salzsäure wird ein pH-Wert von 1 eingestellt. Die wäßrige Phase wird abgetrennt und kann zur Rückgewinnung des (+)-(1 S,2R)-Ephedrins analog Beispiel 15 verwendet werden. Die organische Phase wird mit Wasser gewaschen und im Vakuum eingeengt. Der Rückstand wird zweimal mit Toluol versetzt und zur Trockne eingedampft. Ausbeute: 1.82 g der Titelsäure mit 98 % ee der (S)-Form (HPLC).

### Beispiel 27

### (+)-(1S,2R)-Ephedrin-Salz der (S)-(4-(4-Bromphenyl)-4-methyl-2,5-dioxoimidazolidin-1-yl)essigsäure

6.5 g (R,S)-(4-(4-Bromphenyl)-4-methyl-2,5-dioxoimidazolidin-1-yl)essigsäure und 1.98 g (+)-(1S,2R)-Ephedrin werden in 240 ml Ethanol zum Rückfluß erhitzt. Man läßt langsam zunächst auf 20 bis 25°C abkühlen und rührt dann weitere 2 h bei 0 bis 4°C. Das ausgefallene Produkt wird abfiltriert und getrocknet. Ausbeute: 2.3 g des Titelsalzes mit 70 % ee der (S)-Säure (HPLC).

## Patentansprüche

1. Verfahren zur Herstellung chiraler, nicht racemischer Verbindungen der allgemeinen Formel I, 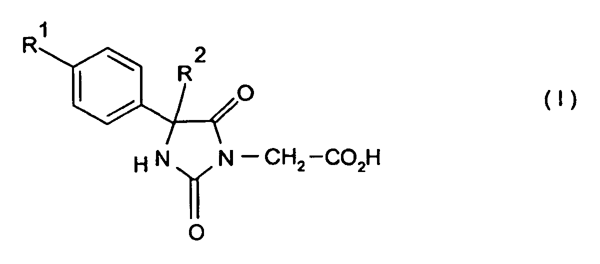 in der
R¹ für Wasserstoff, Fluor, Chlor, Brom, Iod, Cyan, Nitro oder Hydroxy steht und
R² für Wasserstoff, Fluor, (C₁-C₇)-Alkyl, Phenyl-(C₁-C₇)-alkyl oder (C₃₋C₈)-Cycloalkyl steht, **dadurch gekennzeichnet, daß** eine Racematspaltung mit der racemischen Verbindung der allgemeinen Formel I durchgeführt wird, bei der aus der racemischen Verbindung der allgemeinen Formel I und einem chiralen, nicht racemischen phenylsubstituierten Aminoalkohol ein Salz gebildet wird.

2. Verfahren gemäß Anspruch 1, **dadurch gekennzeichnet, daß** R¹ für Chlor, Brom, Iod, Cyan oder Hydroxy steht.

3. Verfahren gemäß Anspruch 1 und/oder 2, **dadurch gekennzeichnet, daß** R² für Wasserstoff, Fluor, (C₁-C₄)-Alkyl, Benzyl oder (C₃-C₇)-Cycloalkyl steht.

4. Verfahren gemäß einem oder mehreren der Ansprüche 1 bis 3, **dadurch gekennzeichnet, daß** R² für Wasserstoff, Methyl oder Ethyl steht.

5. Verfahren gemäß einem oder mehreren der Ansprüche 1 bis 4, **dadurch gekennzeichnet, daß** zur Herstellung der in die Racematspaltung eingesetzten racemischen Verbindungen der allgemeinen Formel I eine Carbonylverbindung der allgemeinen Formel II, 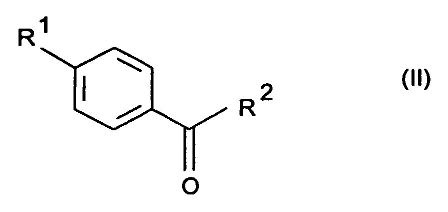 in der
R¹ für Wasserstoff, Fluor, Chlor, Brom, Iod, Cyan, Nitro oder Hydroxy steht und
R² für Wasserstoff, (C₁-C₇)-Alkyl, Phenyl-(C₁-C₇)-alkyl oder (C₃₋C₈)-Cycloalkyl steht, unter den Bedingungen der Bucherer-Reaktion in ein Hydantoin der allgemeinen Formel III, 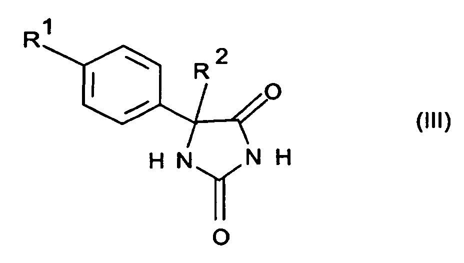 in der R¹ und R² wie für die allgemeine Formel II angegeben definiert sind, überführt wird, dieses mit einem Halogenessigsäureester der allgemeinen Formel IV, 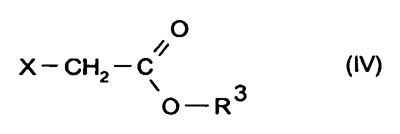 in der X für Chlor, Brom oder Iod steht und R³ für (C₁-C₆)-Alkyl oder Benzyl steht, zu einer Verbindung der allgemeinen Formel V, 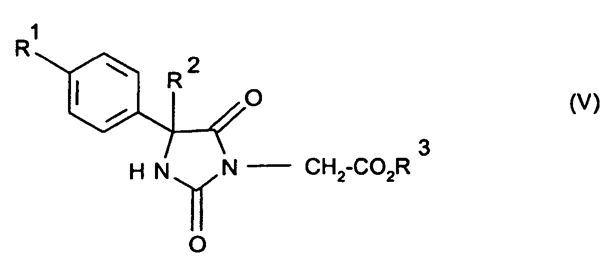 in der R¹ und R² wie für die allgemeine Formel II angegeben definiert sind und R³ für (C₁-C₆)-Alkyl oder Benzyl steht, alkyliert wird, der Ester der allgemeinen Formel V in die Säure der allgemeinen Formel I überführt wird und gewünschtenfalls eine Umwandlung des Substituenten R¹ durchgeführt wird.

6. Verfahren gemäß einem oder mehreren der Ansprüche 1 bis 5, **dadurch gekennzeichnet, daß** als chiraler, nicht racemischer phenylsubstituierter Aminoalkohol (+)-Phenylalaninol oder (-)-Phenylalaninol eingesetzt wird

7. Verfahren gemäß einem oder mehreren der Ansprüche 1 bis 6, **dadurch gekennzeichnet, daß** bei der Salzbildung eine nicht chirale Hilfsbase zugesetzt wird.

8. Verfahren gemäß einem oder mehreren der Ansprüche 1 bis 7, **dadurch gekennzeichnet, daß** die Bildung des Salzes aus der Verbindung der allgemeinen Formel I und dem chiralen, nicht racemischen phenylsubstituierten Aminoalkohol in Wasser, wäßrig-organischen Lösungsmitteln oder Alkoholen durchgeführt wird.

9. Verfahren gemäß einem oder mehreren der Ansprüche 1 bis 8, **dadurch gekennzeichnet, daß** die Bildung des Salzes aus der Verbindung der allgemeinen Formel I und dem chiralen, nicht racemischen phenylsubstituierten Aminoalkohol in Methanol, Ethanol oder Isopropanol durchgeführt wird.

10. Verfahren gemäß einem oder mehreren der Ansprüche 1 bis 9, **dadurch gekennzeichnet, daß** ein aus der Verbindung der allgemeinen Formel I und dem chiralen, nicht racemischen phenylsubstituierten Aminoalkohol gebildetes Salz fraktionierend kristallisiert wird und nach Isolierung gewünschtenfalls anschließend umkristallisiert wird.

11. Verfahren gemäß einem oder mehreren der Ansprüche 1 bis 10, **dadurch gekennzeichnet, daß** aus einem Salz aus der Verbindung der allgemeinen Formel und dem chiralen, nicht racemischen phenylsubstituierten Aminoalkohol durch Behandlung mit einer Mineralsäure die freie Carbonsäure der allgemeinen Formel freigesetzt wird.

12. Verfahren gemäß einem oder mehreren der Ansprüche 1 bis 11, **dadurch gekennzeichnet, daß** (S)-(4-(4-Cyanphenyl)-4-methyl-2,5-dioxoimidazolidin-1-yl)essigsäure hergestellt wird.

13. Verbindungen der allgemeinen Formel Ia, 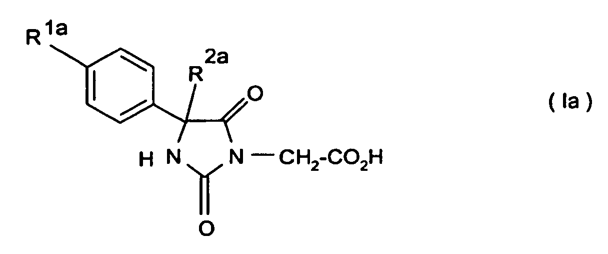 in der
R^{1a} für Fluor, Chlor, Brom oder Hydroxy steht und
R^{2a} für (C₁-C₄)-Alkyl oder Benzyl steht,
in racemischer oder nicht racemischer Form, wobei die in Form der reinen Enantiomeren vorliegende Verbindung der allgemeinen Formel la ausgeschlossen ist, in der R^{1a} für Brom und R^{2a} für Methyl steht, und die in racemischer Form vorliegende Verbindung der allgemeinen Formel la ausgeschlossen ist, in der R^{1a} für Chlor und R^{2a} für Methyl steht.

14. Verbindung der allgemeinen Formel la gemäß Anspruch 13, in der R^{1a} für Brom und R^{2a} für Methyl steht, in racemischer Form.

15. Verbindungen der allgemeinen Formel Va, 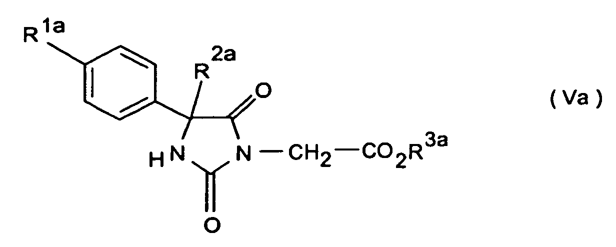 in der
R^{1a} für Fluor, Chlor, Brom oder Hydroxy steht,
R^{2a} für (C₁-C₄)-Alkyl oder Benzyl steht und
R^{3a} für (C₁-C₆)-Alkyl oder Benzyl steht,
in racemischer Form, wobei die Verbindung der allgemeinen Formel Va ausgeschlossen ist, in der R^{1a} für Chlor, R^{2a} für Methyl und R^{3a} für Ethyl steht.

16. Verbindungen der allgemeinen Formel Va gemäß Anspruch 15, in der R^{1a} für Brom, R^{2a} für Methyl und R^{3a} für (C₁-C₄)-Alkyl steht, in racemischer Form.

## Claims

1. A process for the preparation of chiral, nonracemic compounds of the formula I 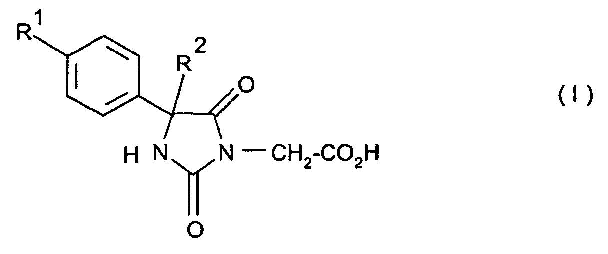 in which
R¹ is hydrogen, fluorine, chlorine, bromine, iodine, cyano, nitro or hydroxyl and
R² is hydrogen, fluorine, (C₁-C₇)-alkyl, phenyl-(C₁-C₇)-alkyl or (C₃-C₈)-cycloalkyl, which comprises carrying out a resolution with the racemic compound of the formula I, in which a salt is formed from the racemic compound of the formula I and a chiral, nonracemic phenyl-substituted aminoalcohol.

2. The process as claimed in claim 1, wherein R¹ is chlorine, bromine, iodine, cyano or hydroxyl.

3. The process as claimed in claim 1 and/or 2, wherein R² is hydrogen, fluorine, (C₁-C₄)-alkyl, benzyl or (C₃-C₇)-cycloalkyl.

4. The process as claimed in one or more of claims 1 to 3, wherein R² is hydrogen, methyl or ethyl.

5. The process as claimed in one or more of claims 1 to 4, wherein, for the preparation of the racemic compounds of the formula I employed in the resolution, a carbonyl compound of the formula II 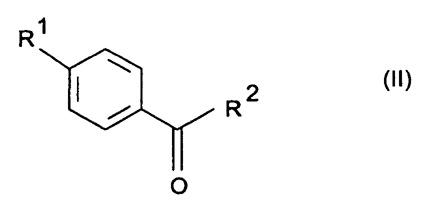 in which
R¹ is hydrogen, fluorine, chlorine, bromine, iodine, cyano, nitro or hydroxyl and
R² is hydrogen, (C₁-C₇)-alkyl, phenyl-(C₁-C₇)-alkyl or (C₃-C₈)-cycloalkyl,
is converted under the conditions of the Bucherer reaction into a hydantoin of the formula III 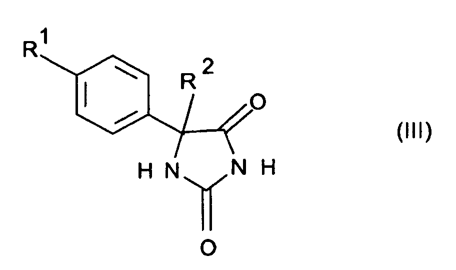 in which R¹ and R² are defined as indicated for the formula II, this is alkylated using a haloacetic acid ester of the formula IV 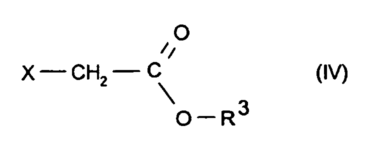 in which X is chlorine, bromine or iodine and R³ is (C₁-C₆)-alkyl or benzyl, to give a compound of the formula V 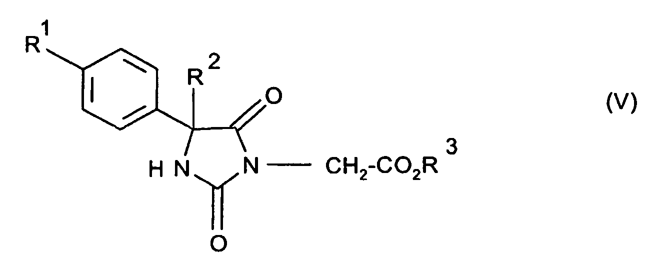 in which R¹ and R² are defined as indicated for the formula II and R³ is (C₁-C₆)-alkyl or benzyl, the ester of the formula V is converted into the acid of the formula I and, if desired, a conversion of the substituent R¹ is carried out.

6. The process as claimed in one or more of claims 1 to 5, wherein the chiral, nonracemic phenyl-substituted aminoalcohol employed is (+)-phenylalaninol or (-)-phenylalaninol.

7. The process as claimed in one or more of claims 1 to 6, wherein a nonchiral auxiliary base is added during salt formation.

8. The process as claimed in one or more of claims 1 to 7, wherein the formation of the salt from the compound of the formula I and the chiral, nonracemic phenyl-substituted aminoalcohol is carried out in water, aqueous-organic solvents or alcohols.

9. The process as claimed in one or more of claims 1 to 8, wherein the formation of the salt from the compound of the formula I and the chiral, nonracemic aminoalcohol is carried out in methanol, ethanol or isopropanol.

10. The process as claimed in one or more of claims 1 to 9, wherein a salt formed from the compound of the formula I and the chiral, nonracemic phenyl-substituted aminoalcohol is fractionally crystallized and, after isolation, is then recrystallized if desired.

11. The process as claimed in one or more of claims 1 to 10, wherein the free carboxylic acid of the formula I is released from a salt of the compound of the formula I and the chiral, nonracemic phenyl-substituted aminoalcohol by treatment with a mineral acid.

12. The process as claimed in one or more of claims 1 to 11, wherein (S)-(4-(4-cyanophenyl)-4-methyl-2,5-dioxoimidazolidin-1-yl)acetic acid is prepared.

13. A compound of the formula la 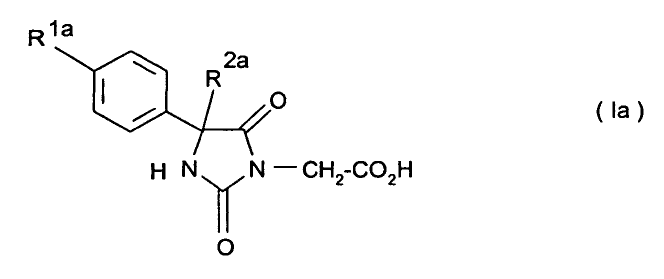 in which
R^{1a} is fluorine, chlorine, bromine or hydroxyl and
R^{2a} is (C₁-C₄)-alkyl or benzyl,
in racemic or nonracemic form, the compound of the formula la present in the form of the pure enantiomers being excluded in which R^{1a} is bromine and R^{2a} is methyl, and the compound of the formula la present in racemic form in which R^{1a} is chlorine and R^{2a} is methyl being excluded.

14. A compound of the formula la as claimed in claim 13, in which R^{1a} is bromine and R^{2a} is methyl, in racemic form.

15. A compound of the formula Va 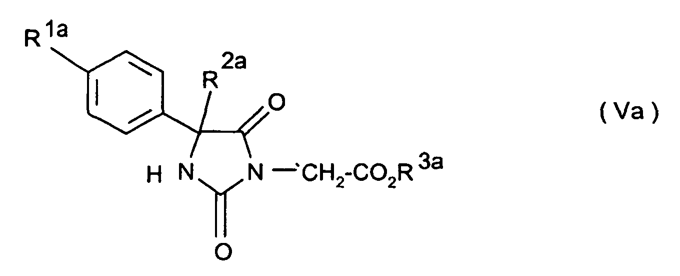 in which
R^{1a} is fluorine, chlorine, bromine or hydroxyl,
R^{2a} is (C₁-C₄)-alkyl or benzyl and
R^{3a} is (C₁-C₆)-alkyl or benzyl,
in racemic form, the compound of the formula Va in which R^{1a} is chlorine, R^{2a} is methyl and R^{3a} is ethyl being excluded.

16. A compound of the formula Va as claimed in claim 15, in which R^{1a} is bromine, R^{2a} is methyl and R^{3a} is (C₁-C₄)-alkyl, in racemic form.

## Revendications

1. Procédé pour la préparation de composés chiraux, non racémiques de formule générale I, 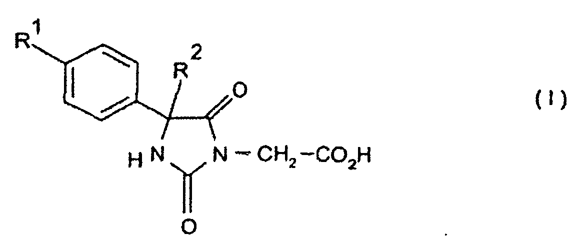 dans laquelle
R¹ représente hydrogène, fluor, chlore, brome, iode, cyano, nitro ou hydroxy et
R² représente hydrogène, fluor, alkyle en C₁ à C₇, phényl (alkyle en C₁ à C₇) ou cycloalkyle en C₃ à C₈, **caractérisé en ce qu'**on réalise une dissociation du racémate avec le composé racémique de formule générale I, lors de laquelle il se forme à partir du composé racémique de formule générale I et un aminoalcool chiral non racémique substitué par phényle, un sel.

2. Procédé selon la revendication 1, **caractérisé en ce que** R¹ représente chlore, brome, iode, cyano ou hydroxy.

3. Procédé selon la revendication 1 et/ou 2, **caractérisé en ce que** R² représente hydrogène, fluor, alkyle en C₁ à C₄, benzyle ou cycloalkyle en C₃ à C₇.

4. Procédé selon l'une ou plusieurs des revendications 1 à 3, **caractérisé en ce que** R² représente hydrogène, méthyle ou éthyle.

5. Procédé selon l'une ou plusieurs des revendications 1 à 4, **caractérisé en ce que**, pour la préparation des composés racémiques de formule générale I, utilisés dans la dissociation du racémate, on transforme un composé carbonyle de formule générale II, 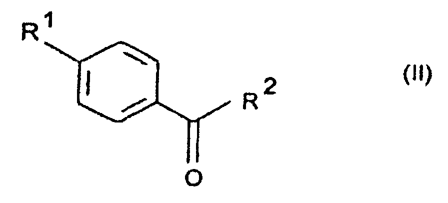 dans laquelle
R¹ représente hydrogène, fluor, chlore, brome, iode, cyano, nitro ou hydroxy et
R² représente hydrogène, alkyle en C₁ à C₇, phényl (alkyle en C₁ à C₇) ou cycloalkyle en C₃ à C₈, dans les conditions de la réaction de Bucherer en une hydantoïne de formule générale III, 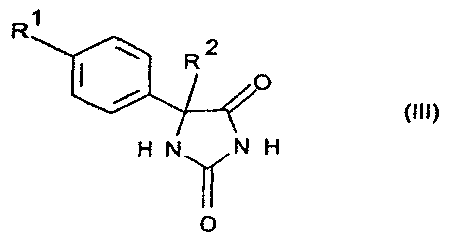 dans laquelle R¹ et R² sont définis comme indiqué pour la formule générale II, on alkyle celle-ci avec un ester d'acide halogénoacétique de formule générale IV, 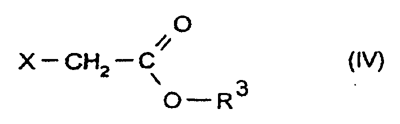 dans laquelle X représente chlore, brome ou iode et R³ représente alkyle en C₁ à C₆ ou benzyle, en un composé de formule générale V, 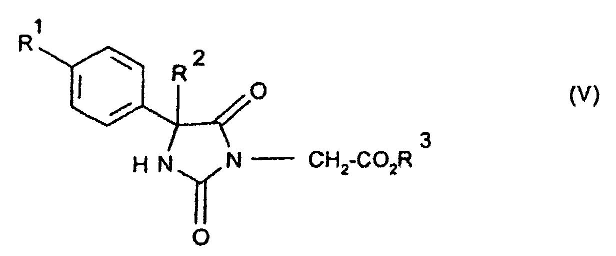 dans laquelle R¹ et R² sont définis comme indiqué pour la formule générale II et R³ représente alkyle en C₁ à C₆ ou benzyle, on transforme l'ester de formule générale V en acide de formule générale I et on réalise si souhaité une transformation du substituant R¹.

6. Procédé selon l'une ou plusieurs des revendications 1 à 5, **caractérisé en ce qu'**on utilise comme aminoalcool chiral, non racémique, substitué par phényle le (+)-phénylalaninol ou le (-)-phénylalaninol.

7. Procédé selon l'une ou plusieurs des revendications 1 à 6, **caractérisé en ce qu'**on ajoute lors de la formation du sel une base auxiliaire non chirale.

8. Procédé selon l'une ou plusieurs des revendications 1 à 7, **caractérisé en ce que** la formation du sel à partir du composé de formule générale I et de l'aminoalcool chiral, non racémique, substitué par phényle est réalisée dans de l'eau, des solvants aqueux-organiques ou des alcools.

9. Procédé selon l'une ou plusieurs des revendications 1 à 8, **caractérisé en ce que** la formation du sel à partir du composé de formule générale I et de l'aminoalcool chiral, non racémique, substitué par phényle est réalisée dans du méthanol, de l'éthanol ou de l'isopropanol.

10. Procédé selon l'une ou plusieurs des revendications 1 à 9, **caractérisé en ce qu'**un sel formé à partir du composé de formule générale I et de l'aminoalcool chiral, non racémique, substitué par phényle est cristallisé par fractionnement et est, si souhaité, recristallisé consécutivement après l'isolement.

11. Procédé selon l'une ou plusieurs des revendications 1 à 10, **caractérisé en ce qu'**on libère à partir d'un sel formé à partir du composé de formule générale I et de l'aminoalcool chiral, non racémique, substitué par phényle l'acide carboxylique libre de formule générale I par traitement avec un acide minéral.

12. Procédé selon l'une ou plusieurs des revendications 1 à 11, **caractérisé en ce qu'**on prépare l'acide (S)-(4-(4-cyanophényl)-4-méthyl-2,5-dioxoimidazolidin-1-yl)acétique.

13. Composés de formule générale Ia 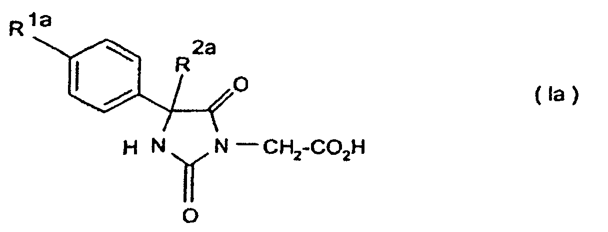 dans laquelle
R^{1a} représente fluor, chlore, brome ou hydroxy et
R^{2a} représente alkyle en C₁ à C₄ ou benzyle,
sous une forme racémique ou non racémique, le composé de formule générale Ia, se trouvant sous forme des énantiomères purs, dans lequel R^{1a} représente brome et R^{2a} représente méthyle étant exclu, et le composé de formule générale Ia, se trouvant sous forme racémique, dans lequel R^{1a} représente chlore et R^{2a} représente méthyle étant exclu.

14. Composé de formule générale Ia selon la revendication 13, dans laquelle R^{1a} représente brome et R^{2a} représente méthyle, sous forme racémique.

15. Composés de formule générale Va, 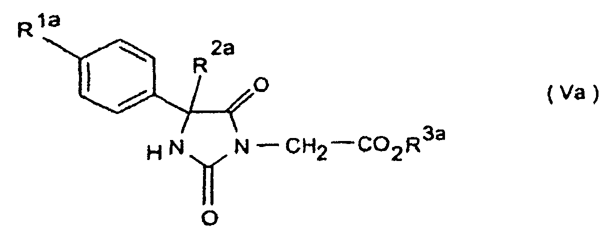 dans laquelle
R^{1a} représente fluor, chlore, brome ou hydroxy,
R^{2a} représente alkyle en C₁ à C₄ ou benzyle et
R^{3a} représente alkyle en C₁ à C₆ ou benzyle,
sous forme racémique, le composé de formule générale Va dans laquelle R^{1a} représente chlore, R^{2a} représente méthyle et R^{3a} représente éthyle étant exclu.

16. Composés de formule générale Va selon la revendication 15, dans laquelle R^{1a} représente brome, R^{2a} représente méthyle et R^{3a} représente alkyle en C₁ à C₄ sous forme racémique.
